## Europäisches Patentamt

## European Patent Office

## Office européen des brevets

(11) Numéro de publication : **0 504 043 A1**

## DEMANDE DE BREVET EUROPEEN

(21) Numéro de dépôt : **92400630.7**

(51) Int. Cl.⁵ : **A61K 7/48,** A61K 7/00

(22) Date de dépôt : **11.03.92**

(30) Priorité : **12.03.91 FR 9102985**

(43) Date de publication de la demande :
**16.09.92 Bulletin 92/38**

(84) Etats contractants désignés :
**AT BE CH DE DK ES FR GB GR IT LI LU NL PT SE**

(71) Demandeur : **ELF SANOFI**
**32-34, rue Marbeuf**
**F-75008 Paris (FR)**

(72) Inventeur : **Maureaud, Thierry**
**61 Les Jardins de Nambours**
**F-31650 Auzielle (FR)**
Inventeur : **Laruel, René**
**35 rue des Pêcheries**
**B-1170 Bruxelles (BE)**
Inventeur : **Sabadie, Michel**
**Rue de la Fontaine Saint-Germain**
**F-27500 Bernay (FR)**

(74) Mandataire : **Gillard, Marie-Louise**
**Cabinet Beau de Loménie 55, Rue d'Amsterdam**
**F-75008 Paris (FR)**

(54) **Composition cosmétique à base de protéine de transfert de phospholipides.**

(57) L'invention concerne des compositions cosmétiques qui ralentissent le vieillissement de la peau, à base de protéines de transfert d'origine végétale ou, de préférence, microbiologique et, éventuellement, de phospholipides ou liposomes.

EP 0 504 043 A1

La présente invention concerne des compositions cosmétiques ayant un effet réparateur sur l'épiderme et, comme conséquence, un effet ralentissant le vieillissement de la peau.

Plus particulièrement, la présente invention se rapporte à des compositions cosmétiques qui ralentissent le vieillissement de la peau, à base de protéines de transfert d'origine végétale ou microbiologique, qui permettent le transfert des phospholipides vers l'épiderme avec un effet réparateur.

Il est bien connu que dans le cytoplasme il existe des protéines, appelées protéines de transfert de membrane et ci-après désignées "protéines de transfert", qui sont capables de transférer des phospholipides d'une membrane à une autre. Cette propriété a été mise en évidence pour la première fois par Wirtz et al., J. Biol. Chem., 1968, 243, 3956-3602, qui ont observé le phénomène du transfert des phospholipides entre des microsomes et des mitochondries de foie de rat.

De nombreuses protéines ont été purifiées et caractérisées à partir de différents tissus animaux ou végétaux, ainsi qu'à partir de cellules procaryotiques ou de micro-organismes eucaryotiques.

Les phospholipides qui sont transférés d'une membrane à l'autre grâce à l'action des protéines de transfert sont notamment la phosphatidylcholine, la phosphatidyléthanolamine et le phosphatidylinositol. La protéine de transfert peut être spécifique, pour le transport d'un seul phospholipide, ou aspécifique, favorisant ainsi le transport de deux ou de tous les phospholipides membranaires.

Il est également connu que l'action desdites protéines sur le transfert des phospholipides a lieu également in vitro à partir des phospholipides contenus dans des membranes naturelles, par exemple celles des érythrocytes humains ou celles des micro-organismes eucaryotiques ou procaryotiques, ou dans des membranes artificielles, par exemple les liposomes, synthétiques ou naturels.

Sur la base de ces connaissances, la demande de brevet français 2 631236 envisage l'utilisation de "systèmes actifs" contenant des protéines de transfert et des composants lipophiliques ou amphiphiliques pour l'échange de lipides entre ces systèmes et des structures cibles, dans différents domaines tels que la technologie des matériaux, la médecine, la pharmacie, et la cosmétique.

La description générale contenue dans cette demande de brevet n'est pas suffisante pour pouvoir exécuter une quelconque de ces utilisations. Elle ne contient aucune description détaillée des protéines utilisables (certaines d'entre elles ont des problèmes de stabilité et/ou de contamination),ni de la manière de les obtenir et de les utiliser (quantité, activité spécifique, etc.) dans chaque type d'utilisation suggérée. De plus, étant donné que, selon cette demande de brevet, l'échange peut se produire dans les deux sens, tant du système actif à la structure cible que de la structure cible au système actif, on ne peut pas déduire comment obtenir l'un ou l'autre.

On a maintenant trouvé qu'on peut obtenir une composition cosmétique stable qui comprend une quantité efficace du point de vue cosmétique d'une protéine de transfert d'origine particulière et que, lorsqu'on l'applique sur la peau, celle-ci provoque un enrichissement des cellules de l'épiderme en phospholipides. Lesdits phospholipides peuvent être ceux déjà présents au niveau de la peau ou bien ils peuvent être associés, tels quels ou sous forme de liposomes, à la protéine de transfert. Cet enrichissement comporte un effet réparateur ralentissant le vieillissement cutané.

La protéine de transfert peut être d'origine végétale ou, de préférence, issue de micro-organismes et peut être utilisée à l'état pur ou, de préférence, en solution après purification éventuelle.

Ainsi, la présente invention concerne des compositions cosmétiques contenant au moins une protéine de transfert d'origine végétale ou microbiologique, dans un excipient cosmétique.

Parmi les protéines de transfert d'origine végétale, éventuellement chimiquement modifiées, on peut citer par exemple :

– les protéines extraites de graines de maïs (Zea mays) comme celle décrite par
    . Douday et al., Biochim. Biophys. Acta 1982, 710, 143-153 ;
– les protéines extraites du ricin comme celle décrite par
    . Watanabe et al., Biochim. Biophys. Acta 1986, 876, 116-123; et caractérisée par
    . Takishima et al., Biochim. Biophys. Acta 1986, 870, 248-255 ;
– les protéines extraites des épinards (Spinacia oleracea L.) comme celle décrite par
    . Kader et al., Eur. J. Biochem. 1984, 139, 411-416
    . Julienne et al., C.R. Acad. Sc. Paris 1983, 296, Série III, 617-620 et caractérisée par
    . Bouillon et al., Eur. J. Biochem. 1987, 166, 387-391. ou également celles provenant de champignons, par exemple
– les protéines extraites de champignons filamenteux comme celle obtenue de Mucor mucedo décrite par
    – Chavant et al., Biochemistry and Metabolism of Plant lipids, 1982, Ed. J.F.G.M. Wintermans, P.J.C. Kuiper, Elsevier Biomedical Press B.V., 125-128.

Selon un aspect préféré de la présente invention, d'autres protéines de transfert qui peuvent être utilisées sont celles provenant de micro-organismes, notamment de bactéries et de levures, par exemple

– les protéines présentes dans les extraits de <u>Bacillus subtilis</u> comme celles décrites par

. Frehel et al., Biochim. Biophys. Acta 1970, 223, 292

. Bureau et al., FEBS Letters 1974, 39, 332-336 ;

– les protéines présentes dans des extraits de <u>Rhodopseudomonas sphaeroides</u> obtenus comme décrit par

. Cohen et al., J. Biol. Chem. 1979, 254, 721-728 ;

– les protéines présentes dans des extraits de <u>Saccharomyces cerevisiae</u> obtenues comme décrit par

. Cobon et al., Biochim. Biophys. Acta 1976, 441, 255-259

. Daum et al., Biochim. Biophys. Acta 1984, 794, 385-391

. Bozzato et al., Biochem. Cell Biol. 1987, 65, 195-202.

Ces protéines de transfert sont préparées selon des méthodes classiques de la littérature telles que celles décrites dans les publications citées ci-dessus.

En général, on arrive à un mélange protéique qui contient la protéine de transfert désirée dans des proportions différentes. Si nécessaire ou convenable, la préparation ainsi obtenue est purifiée de façon plus ou moins poussée en donnant des préparations enrichies en protéine de transfert qui, à leur tour, peuvent être purifiées ultérieurement jusqu'à l'obtention de préparations constituées essentiellement de protéine de transfert.

L'activité spécifique d'une quelconque de ces préparations, contenant une protéine de transfert, est convenablement exprimée comme l'activité de transfert des phospholipides, en nmoles de phospholipides transférés par heure, rapportée à la quantité de protéine totale de la préparation. Cette activité de transfert de phospholipides est analysée selon un dosage qui met en évidence le transfert de phospholipides entre une membrane donneuse (constituée par des liposomes unilamellaires obtenus par extrusion à partir de phosphatidylcholine d'oeuf contenant un lipide neutre marqué avec du $^{14}$C et un phospholipide marqué avec du $^3$H) et une membrane receveuse constituée par des mitochondries de foie de rat. Le test consiste à incuber à 30°C pendant 30 minutes l'échantillon à analyser en présence des deux types de membranes, éliminer les membranes donneuses par centrifugation et évaluer le pourcentage de phospholipides transférés dans les membranes receveuses par comptage de la radioactivité due au tritium. La mesure de la radioactivité due au $^{14}$C sert à évaluer le transfert non spécifique et la contamination résiduelle des membranes receveuses par les membranes donneuses.

L'activité de transfert dans l'échantillon est exprimée en nmoles de phospholipides transférés par heure et l'activité spécifique de l'échantillon est l'activité de transfet rapportée à la quantité de protéines totales dans l'échantillon.

Les compositions cosmétiques selon la présente invention comprennent au moins une protéine de transfert d'origine végétale ou microbiologique dans des proportions, en pourcentage poids/poids, qui dépendent de l'activité spécifique de la préparation contenant la protéine qu'on utilise. Plus l'activité spécifique de la préparation de protéine de transfert utilisée est élevée, plus la quantité, en poids, nécessaire pour obtenir l'effet réparateur désiré est faible, et vice versa.

Pour obtenir des compositions cosmétiques selon l'invention, on peut convenablement utiliser des préparations de protéines de transfert ayant une activité spécifique aussi faible que par exemple 25 nM/mg/h, une activité spécifique d'au moins environ 250 nM/mg/h étant préférée.

En pratique, les compositions cosmétiques de la présente invention comprennent au moins une protéine de transfert d'origine végétale ou microbiologique en proportions comprises entre 0,001 et 10 % en poids, et de préférence entre 0,001 et 5 % en poids.

De préférence, les protéines de transfert des compositions cosmétiques de la présente invention sont celles provenant de micro-organismes, notamment de levures.

Les levures, notamment <u>Saccharomyces cerevisiae</u>, sont cultivées comme décrit dans la littérature et l'extrait contenant la protéine de transfert est obtenu de préférence par choc osmotique ou en utilisant un désintégrateur aux ultrasons. N'importe quelle souche de <u>Saccharomyces cerevisiae</u> peut être employée pour obtenir des protéines de transfert de phospholipides utilisables dans les compositions cosmétiques de la présente invention, tant des souches sauvages que des souches génétiquement modifiées. En plus des souches déposées dans les différentes collections, telles que l'ATCC, la NRRL, la CBS, etc., il faut y inclure aussi celles qui sont toujours disponibles et quotidiennement utilisées, par exemple sous le nom de levure de boulanger.

Selon un des aspects préférentiels de l'invention, les protéines de transfert sont associées, dans les compositions cosmétiques de la présente invention, à un ou plusieurs phospholipides. Les phospholipides qui peuvent être associés aux protéines de transfert dans les compositions selon l'invention comprennent notamment la phosphatidylcholine, la phosphatidyléthanolamine et le phosphatidylinositol.

Selon un aspect encore plus avantageux, les protéines de transfert sont associées dans les compositions de l'invention à un ou plusieurs phospholipides sous forme de liposomes ou de mélange de liposomes.

Les liposomes sont des vésicules lipidiques bilamellaires, rappelant la structure des membranes cellulaires, qui peuvent être formées à partir de divers phospholipides.

Des substances liposolubles peuvent être fixées au niveau des couches lipidiques, et des substances solubles dans l'eau dans les couches aqueuses.

Différents phospholipides peuvent être utilisés et, selon les cas, les liposomes peuvent être solides ou liquides. Ces phospholipides sont de préférence :

– la phosphatidyléthanolamine,
– la lécithine d'oeuf (phosphatidylcholine), éventuellement en mélange avec la phosphatidylsérine,
– le phosphaditylinositol,
– la dipalmitoylphosphatidylcholine.

Ces phospholipides, auxquels on ajoute du cholestérol, sont dissous dans le chloroforme, lequel est ensuite évaporé par rotation sous pression réduite.

La charge électrique des liposomes peut être modifiée par l'adjonction de certaines substances : acide phosphatidique (charge négative), stéarylamine (charge positive). En l'absence de ces substances, les vésicules sont neutres.

Par liposome, on entend toute vésicule phospholipidique naturelle ou synthétique, neutre, basique ou acide telle que décrite ci-dessus et dans la littérature (Liposomes, Ed. M. Ostro, Marcel Dekker, New York, 1983).

Selon le nombre de leurs compartiments, leur taille et leur mode d'obtention, on peut distinguer au moins quatre types de liposomes :

– les vésicules multilamellaires, qui présentent plusieurs compartiments aqueux concentriques et dont le diamètre peut atteindre de 0,1 à 2 $\mu$m ; elles sont généralement préparées par déposition d'une solution organique des lipides sous forme d'un film suivie par l'hydratation des lipides en les dispersant dans une solution aqueuse tamponnée ;

– les petites vésicules unilamellaires, qui comportent une seule paroi et une seule cavité aqueuse et ont un diamètre de l'ordre de 25 nm ; elles peuvent être obtenues par l'action des ultrasons (sonication), par injection rapide d'une solution éthanolique des phospholipides dans l'eau ou dans une solution tamponnée, par l'élimination de détergent à partir de micelles mixtes détergent/phospholipide, par extrusion à haute pression à travers la presse de French, ou encore par formation spontanée à partir de dispersions contenant les acides phosphatidiques, en augmentant rapidement le pH de la dispersion ;

– les grosses vésicules unilamellaires, qui sont des vésicules ayant un diamètre de 0,2 à 1 $\mu$m, mais entourées d'une seule bicouche lipidique ; elles peuvent être préparées par infusion dans l'éther ou par fusion induite par le calcium, selon des techniques bien connues sous ces dénominations, ou par leurs modifications également connues ; et

– les vésicules obtenues par évaporation de phase, qui sont de grosses vésicules unilamellaires obtenues en deux étapes : par dispersion d'une solution organique d'un phospholipide avec formation d'une émulsion, suivie par l'évaporation du solvant sous pression réduite.

Tous ces types de liposomes sont appropriés pour l'utilisation dans les compositions cosmétiques de la présente invention.

Les phospholipides ou les liposomes à associer aux protéines de transfert dans les compositions cosmétiques de la présente invention peuvent être choisis de façon à faire correspondre le phospholipide ou le phospholipide utilisé pour la préparation des liposomes avec la capacité de transfert des protéines. Ainsi, par exemple, lorsqu'on utilise de la phosphatidylcholine ou des liposomes à base de phosphatidylcholine, il peut être utile de leur associer une protéine de transfert spécifique pour la phosphatidylcholine. Par contre, lorsqu'on utilise un mélange de différents phospholipides ou des liposomes à base de différents phospholipides, on peut leur associer une protéine de transfert spécifique ou plusieurs protéines de transfert spécifiques pour chaque phospholipide présent.

Lorsque, selon un aspect préféré de la présente invention, les protéines de transfert sont associées à un ou plusieurs phospholipides, lesdits phospholipides sont généralement présents dans des proportions comprises entre 0,001 et 50 % et de préférence entre 0,01 et 20 %.

Lorsque, selon un aspect encore plus préféré, les phospholipides associés aux protéines de transfert sont sous forme de liposomes ou de mélanges de liposomes, ils sont contenus dans les compositions de la présente invention dans des proportions comprises entre 0,01 et 50 % et, de préférence, entre 1 et 20 % en poids.

Elles peuvent contenir de l'eau et/ou des solvants ou des diluants compatibles avec la peau ainsi qu'avec les ingrédients actifs de la composition même.

Ces compositions contiennent habituellement des excipients ou des additifs choisis parmi les ingrédients habituellement utilisés dans des compositions destinées à une application topique, selon la nécessité des formulations particulières envisagées.

Elles peuvent contenir par exemple des agents épaississants, des mouillants, des adoucissants, des surgraissants, des émollients, des stabilisants, des agents anti-mousse, des tensioactifs, des filtres solaires, des antioxydants, des colorants et/ou des pigments, et des parfums. Evidemment, lorsque les compositions cosmétiques de la présente invention contiennent des liposomes, il faudra choisir les additifs, en particulier les tensioactifs, de façon qu'ils soient compatibles avec les liposomes mêmes.

Elles peuvent également contenir d'autres substances actives ayant un effet sur le vieillissement de la peau soit un système de protection ou d'inhibition des agressions extérieures (par exemple un produit filmogène ou un agent antiradicalaire), soit un mécanisme de régénération et/ou restructuration de l'épiderme (des stimulateurs de la synthèse du collagène, des inhibiteurs de la collagénase ou de l'élastase).

La forme des compositions cosmétiques selon l'invention peut être une crème dans laquelle le constituant ou le mélange de constituants, avec un stabilisant éventuel, est associé aux excipients couramment utilisés dans la cosmétologie et compatibles avec lesdits constituants, tels que la lanoline ou les huiles végétales, minérales ou synthétiques.

Les compositions cosmétiques de l'invention peuvent être également présentées sous forme de gel dans les excipients appropriés tels que les esters de cellulose ou d'autres agents gélifiants comme les dérivés acryliques.

Les compositions cosmétiques suivant l'invention peuvent aussi prendre la forme d'une lotion ou d'une solution dans laquelle le mélange des constituants est dissous ou microdispersé.

La forme des compositions cosmétiques suivant l'invention peut donc être une microdispersion dans un liquide contenant de l'eau, ainsi qu'un ou plusieurs agents tensioactifs compatibles, éventuellement avec les liposomes. Ces dispersions présentent les caractères des microémulsions et ont pratiquement l'aspect de solutions vraies. Elles peuvent également être préparées extemporanément.

Ces compositions jouissent d'une bonne stabilité et peuvent être conservées pendant le temps nécessaire pour l'utilisation à des températures comprises entre 0 et 60°C sans qu'il y ait sédimentation des constituants ou séparation de phases.

Les tensioactifs de la composition sont choisis parmi les agents de surface utilisables en cosmétologie, et, le cas échéant, compatibles en général avec les liposomes à faible concentration.

Lorsque l'on souhaite maintenir le pH à une valeur voisine de la neutralité, on peut ajouter à la composition un agent de neutralisation. L'agent neutralisant éventuel peut être constitué par un mélange tampon par exemple un tampon phosphate, ou tout simplement par une amine biocompatible telle que la mono-, la di- ou la triéthanolamine.

Les compositions cosmétiques de la présente invention sont très bien tolérées, elles ne présentent aucune phototoxicité et leur application sur la peau pour des périodes de temps prolongées n'implique aucun effet secondaire. En général, après trente jours d'application, on note une amélioration de l'aspect de la peau et un ralentissement dans la formation des rides.

Les exemples suivants illustrent l'invention sans toutefois la limiter.

EXEMPLES D'OBTENTION DES PROTEINES DE TRANSFERT

Exemples 1 à 3

On suit essentiellement le même protocole, qui est décrit ci-dessous, pour la préparation de solutions enrichies en protéines de transfert de phospholipides à partir de souches de la levure Saccharomyces cerevisiae et pour la mise en évidence de cette activité.

– Protocole d'obtention de la biomasse

(a) Fermentation

La biomasse de Saccharomyces cerevisiae est obtenue par un procédé classique de culture en "fed-batch".

A partir d'un lot de semence, on multiplie les cellules dans un erlenmeyer contenant du milieu YNBG (yeast Nitrogen Base + Glucose). On incube cette préculture à 30°C sous agitation pendant environ 24 heures. La préculture sert à incuber, à un taux de 5 % (v/v), un fermenteur Biolafitte équipé de 3 turbines Rushton, contenant du milieu nutritif de Kappeli. La culture est conduite pendant 60 à 70 heures avec les points de consigne suivants :

– température : 30°C
– agitation : 150 à 350 rpm en fonction de la biomasse
– pression : 0,4 bar
– pH : 4
– p02 : 50 % de la saturation.

L'apport régulièrement contrôlé (fed-batch) de milieu nutritif comprenant du glucose permet d'obtenir une concentration en biomasse d'environ 60 g de matière sèche par litre.

– Protocole d'extraction

(b) Récupération et lavage des cellules

Les cellules sont alors récupérées par centrifugation à 5 000 g pendant 10 minutes dans une centrifugeuse Beckman J 2.21 réfrigérée à 4°C.

Après élimination du milieu de culture, on lave la biomasse par une série de centrifugations et de resuspensions dans de l'eau glacée, puis dans du tampon d'extraction : tris (i.e. tris (hydroxyméthyl)-aminométhane)/HCl, pH 7,5, 100 mM contenant 1 mM d'EDTA (acide éthylènediaminotétraacétique), 400 mM de saccharose, 8 mM de 2-mercaptoéthanol et 4 mM de chlorhydrate de cystéine.

(c) Broyage

Après ajustement de la concentration en matière sèche à environ 15 g/l, les cellules sont cassées en continu dans un broyeur à billes Dyno Mill Kds, réfrigéré à 4°C par circulation d'eau glycolée, équipé d'une cellule de 0,6 l de volume utile contenant 510 ml de billes de 0,25-0,5 mm de diamètre, préalablement équilibrée dans du tampon d'extraction. Le temps de séjour moyen dans la cellule est compris entre 4 minutes et 10 minutes, le volume traité est de 290 ml à 800 ml de cellules lavées concentrées à 15 % (P/V) de matière sèche, les cellules cassées sortant du broyeur sont récoltées dans une éprouvette maintenue dans un bain de glace fondante. Le pH du broyat est ajusté à 7,5 avec du tris 1M.

(d) Clarification

Le broyat obtenu est clarifié par centrifugation à 10 000 g pendant 20 minutes dans une centrifugeuse Beckman J 2.21 réfrigérée à 4°C, équipée d'un rotor JA 10. Le surnageant obtenu est soumis à une deuxième centrifugation à 45 000 g pendant 20 minutes ou à 12 000 g pendant 2 heures dans une centrifugeuse Jouan PR 20 réfrigérée à 4°C, équipée d'un rotor 6 x 250 ml.

(e) Précipitation

Le surnageant clarifié maintenu à 4°C est soumis à une précipitation à pH 5,1 par ajout d'acide chlorhydrique à 37 %. Après élimination du précipité par centrifugation et réajustement du pH à 7,5 avec du tris 1M, le surnageant est soumis à une deuxième précipitation avec du sulfate d'ammonium à 60 % de saturation.

Alternativement, le surnageant clarifié est soumis à précipitation directement avec du sulfate d'ammonium à 60 % de saturation sans précipitation acide préalable.

(f) Purification

Les précipités obtenus sont récupérés après élimination des surnageants et remis en suspension dans du tampon de chromatographie : tris/HCl, pH 7,5, 20 mM contenant 2 % de glycérol et contenant ou non 8 mM de 2-mercapto-éthanol, de façon à obtenir une concentration en protéines comprise entre 20 et 30 mg/ml. Les précipités repris sont ensuite filtrés sur filtre 0,45 μm (Sartorius).

Des volumes de 50 à 75 ml de la solution obtenue sont ensuite injectés sur une colonne de chromatographie d'exclusion remplie avec du gel Sephacryl S 200 HR (Pharmacia) de 1,6 l de volume ($\varnothing$ = 50 mm, h = 81,5 cm), préalablement équilibrée avec le tampon de chromatographie. Les effluents de la colonne sont récoltés en fractions de 15 ml.

La concentration en protéines à chaque étape de l'extraction est évaluée selon la méthode de Bradford en utilisant un kit de dosage Biorad avec la sérumalbumine bovine comme standard.

Pour l'analyse de l'activité de transfert de phospholipides dans les fractions issues de la chromatographie d'exclusion on constitue des pools des fractions les plus riches en activité.

Exemple 1

Dans les étapes d'extraction des protéines de transfert, on utilise, comme produit de départ, 290 ml de cellules lavées avec une concentration en matière sèche de 15 %. Après l'étape de broyage, caractérisée par un temps de séjour dans le broyeur de 4 minutes, on obtient 700 ml de broyat contenant 5,65 g de protéines totales, correspondant à 131 mg de protéines extraites par g de matière sèche. On clarifie le broyat en conduisant la deuxième centrifugation à 45 00 g pendant 20 minutes, ce qui porte à 540 ml de surnageant contenant 3,2 g de protéines totales. On soumet le surnageant clarifié à une précipitation directe avec du sulfate de sodium et on obtient 2,1 g de protéines qui sont purifiées par chromatographie d'exclusion comme décrit dans le protocole en mettant en suspension 1,6 g de protéines dans 55 ml de tampon de chromatographie tris/HCl, 20 mM, pH 7,5, contenant 2 % de glycérol et 8 mM de 2-mercaptoéthanol, et filtrant sur filtre 0,45 μm.

On obtient ainsi deux pools:

– PT1 contenant 162 mg de protéines totales ayant une activité spécifique de 1500 nM/mg/h, et
– PT2 contenant 12 mg de protéines totales ayant une activité spécifique de 15 700 nM/mg/h.

Exemple 2

On soumet à broyage, pendant 4 min 30 s, 800 ml de cellules lavées avec une concentration en matière sèche de 15 %. On obtient 1 050 ml de broyat contenant 25,2 g de protéines totales, correspondant à 200 mg de protéines extraites par g de matière sèche, et on le clarifie selon le protocole en conduisant la deuxième centrifugation à 12 000 g pendant, 2 h. On obtient ainsi 565 ml de surnageant clarifié, contenant 11,3 g de protéines totales, qui est soumis à une précipitation en deux étapes comme décrit au point (e) du protocole, donnant 4,03 g de protéines totales.

On purifie une partie des protéines ainsi obtenues par chromatographie d'exclusion comme décrit dans le protocole en mettant en suspension 1,8 g de protéines dans 75 ml de tampon de chromatographie tris/HCl, 20 mM, pH 7,5, contenant 2 % de glycérol et 8 mM de 2-mercaptoéthanol, et en filtrant sur filtre 0,45 μm.

On obtient ainsi un pool:
– PT3 contenant 875 mg de protéines totales ayant une activité spécifique de 920 nM/mg/h.

Exemple 3

On purifie une autre partie des protéines précipitées, obtenues dans l'Exemple 2, par chromatographie d'exclusion en suspendant 1,15 g de protéines dans 50 ml de tampon de chromatographie tris/HCl, 20 mM, pH 7,5, contenant 2 % de glycérol, et en filtrant sur filtre 0,45 μm.

On obtient ainsi un pool:
– PT4 contenant 440 mg de protéines totales ayant une activité spécifique de 1500 nM/mg/h.

Les fractions obtenues sont stables et peuvent donc être utilisées pour la préparation de crèmes ou lotions.

On a étudié leur stabilité à -20°C, +8°C, +20°C et l'activité de transfert a été mesurée régulièrement. Afin d'éviter toute contamination microbienne, on peut ajouter dans les tampons de chromatographie de l'azide de sodium (0,2 %).

Aucune perte d'activité de transfert des phospholipides n'est observée pendant au moins 7 semaines aux températures de -20°C, +8°C et +20°C dans les préparations obtenues dans les Exemples 1 et 2 et pendant au moins une semaine dans la préparation obtenue dans l'Exemple 3.

EXEMPLES DE PREPARATION DES COMPOSITIONS COSMETIQUES

Dans un souci de simplification, certains constituants des compositions ont été désignés par leur dénomination commerciale dont voici les significations :

Acrysol : copolymère acrylates/méthacrylates, commercialisé par SEPPIC

Finsolv TN : benzoates d'alcools contenant 12–15 atomes de carbone, commercialisé par WITCO

Solutol HS 15 : 12–hydroxystéarate de polyéthylèneglycol 600, commercialisé par BASF

Cetiol HE : polyéthylèneglycol–7 glycéryl cocoate, commercialisé par HENKEL

Labrafac hydrophile : triglycérides contenant 7–8 atomes de carbone polyoxy-éthylénés, commercialisé par GATTEFOSSE

Abil 8851 B : diméthicone copolyol, commercialisé par GOLDSCHMIDT

Carbopol 934 )
Carbopol 940 ) carboxypolyméthylène, commercialisé par GOODRICH

EDTA : acide éthylènediaminotétracétique

Filtre UVB : 4–méthoxycinnamate d'éthyle–2 hexyle (marque PARSOL
MCX), commercialisé par GIVAUDAN

Sepicide HB : mélange de conservateurs (phénoxyéthanol esters de l'acide
parahydroxybenzoïque) commercialisé par SEPPIC

Pemulen TR2 : acrylate $C_{10}/C_{30}$–alkyl acrylate cross polymer commercialisé
par GOODRICH

Sepigel 305 : mélange de polyacrylamide, d'isoparaffine et de dodécanol
éthoxyle, commercialisé par SEPPIC

Hypan SA–100H : copolymère d'acrylamide neutralisé par la triéthanolamine,
commercialisé par LIPO CHEMICALS

Coviox T50 : mélange d'isomères de tocophérols commercialisé par HENKEL.

Exemple 4

On prépare un gel traitant aux liposomes en utilisant les composés suivants :

| | | |
|---|---|---|
| eau déminéralisée | q.s.p. | 100, 000 |
| glycérine | | 3,000 |
| hyaluronate de sodium | | 0,020 |
| Carbopol 940 | | 0,500 |
| Polyglycérylméthacrylate | | 5,000 |
| Triéthanolamine | | 0,500 |
| Butylèneglycol | | 5,000 |
| Parfum | | 0,100 |
| Imidazolidinyl urée | | 0,500 |
| Parahydroxybenzoate de méthyle | | 0,200 |
| Liposomes de phosphatidyléthanolamine | | 10,000 |
| Volume de la préparation de protéine de transfert de l'Exemple 2 (PT3) contenant une quantité de protéines totales de | | 1,000 |

Exemple 5

On prépare la composition suivante, utilisable sous forme de lait fluide :

| Eau | qsp | 100,000 |
|---|---|---|
| Butylèneglycol | | 3,500 |
| Palmitate d'éthyl-2-hexyle | | 3,000 |
| Finsolv TN | | 3,000 |
| Propylèneglycol dicaprate dicaprylate | | 3,000 |
| Huile de vaseline | | 3,000 |
| Pemulen TR2 | | 0,200 |
| Triéthanolamine | | 0,200 |
| Sepicide HB | | 0,500 |
| Parfum | | 0,200 |
| EDTA | | 0,100 |
| Liposomes de phosphatidyléthanolamine | | 5,000 |
| Volume de la préparation de protéine de transfert de l'Exemple 3 (PT4) contenant une quantité de protéines totales de | | 0,500 |

Exemple 6

On prépare la composition suivante, utilisable en tant qu'émulsion gel anti-âge :

| Eau | q.s.p. | 100,000 |
|---|---|---|
| EDTA tétrasodique | | 0,100 |
| Hypan SA – 100H | | 0,200 |
| Huile de vaseline | | 10,000 |
| Palmitate d'éthyl-2-hexyle | | 10,000 |
| Dipélargonate de propylèneglycol | | 10,000 |
| Pemulen TR2 | | 0,250 |
| Triéthanolamine | | 0,600 |
| Sepigel 305 | | 2,000 |
| Butylèneglycol | | 5,000 |
| Sepicide HB | | 0,500 |
| Coviox T 50 | | 0,050 |
| Liposomes de phosphatidyléthanolamine | | 5,000 |
| Volume de la préparation de protéine de transfert de l'Exemple 1 (PT2) contenant une quantité de protéines totales de | | 0,500 |

Exemple 7

On prépare la composition suivante, utilisable en tant qu'émulsion gel protectrice :

| | | |
|---|---|---|
| Eau | q.s.p. | 100,000 |
| EDTA trisodique | | 0,100 |
| Butylèneglycol | | 5,000 |
| Sepicide HB | | 0,500 |
| Sepigel 305 | | 3,000 |
| $C_{12}$–$C_{14}$ alcool lactate | | 2,000 |
| Monolaurate de sorbitan éthoxylé | | 0,500 |
| Coviox T 50 | | 0,500 |
| Parsol MCX | | 2,000 |
| Liposomes de phosphatidylcholine | | 10,000 |
| Volume de la préparation de protéine de transfert de l'Exemple 1 (PT1) contenant une quantité de protéines totales de | | 0,500 |

## Exemple 8

On prépare la composition suivante, utilisable comme crème de nuit :

| | | |
|---|---|---|
| Eau déminéralisée | q.s.p. | 100,000 |
| Butylèneglycol | | 5,000 |
| Palmitate d'isopropyle | | 5,000 |
| Alcool cétylique | | 2,000 |
| Stéarine TP | | 2,500 |
| Huiles végétales | | 3,000 |
| Monostéarate de glycérol | | 5,000 |
| Huile de vaseline | | 4,000 |
| Huile de silicone | | 0,500 |
| Triglycérides végétaux | | 2,000 |
| Conservateurs | | 1,000 |
| Triéthanolamine | | 1,000 |
| EDTA tétrasodique | | 0,100 |
| Phosphatidylcholine | | 1,000 |
| Volume de la préparation de protéine de transfert de l'Exemple 2 (PT3) contenant une quantité de protéines totales de | | 1,000 |

## Exemple 9

On prépare la composition suivante, utilisable comme base de maquillage :

| | | |
|---|---|---|
| Eau déminéralisée | q.s.p. | 100,000 |
| Stérols de soja éthoxylés | | 4,000 |
| Stérols de soja | | 0,500 |
| Monostéarate de glycérol | | 1,000 |
| Huile végétale | | 1,500 |
| Palmitate d'éthyl–2–hexyle | | 4,000 |
| Alcool cétylique | | 0,500 |
| Triglycérides d'acides gras courts | | 1,500 |
| Huile de vaseline | | 1,800 |
| Huile de silicone | | 1,000 |
| Alcools de lanoline | | 0,200 |
| Dipélargonate de propylèneglycol | | 3,000 |
| Propylèneglycol | | 4,000 |
| Conservateurs | | 1,000 |
| Carbopol 941 | | 0,200 |
| Triéthanolamine | | 0,200 |
| alpha–tocophérol | | 0,020 |
| EDTA tétrasodique | | 0,100 |
| Parfum | | 0,200 |
| Phosphatidylcholine | | 0,100 |
| Volume de la préparation de protéine de transfert de l'Exemple 3 (PT4) contenant une quantité de protéines totales de | | 1,000 |

Exemple 10

En utilisant les composés suivants :

| | | |
|---|---|---|
| Eau déminéralisée | q.s.p. | 100,000 |
| Solutol HS 15 | | 1,000 |
| Labraphac hydrophile | | 0,250 |
| Cetiol HE | | 0,200 |
| Abil 8851 | | 0,050 |

| | |
|---|---|
| Propylèneglycol | 12,500 |
| Ethanol | 12,500 |
| Carbopol 940 | 0,400 |
| Parfum | 0,300 |
| Conservateurs | 0,500 |
| Triéthanolamine | 0,400 |
| Volume de la préparation de protéine de transfert de l'Exemple 1 (PT2) contenant une quantité de protéines totales de | 0,100 |

on obtient une composition sous forme de microémulsion.

## Revendications

1. Composition cosmétique caractérisée en ce qu'elle comprend au moins une protéine de transfert de phospholipides d'origine végétale ou microbiologique dans un excipient cosmétique.

2. Composition selon la revendication 1, caractérisée en ce que, comme protéine de transfert de phospholipides, on utilise une protéine d'origine microbiologique.

3. Composition selon la revendication 2, caractérisée en ce que, comme protéine de transfert de phospholipides, on utilise une protéine extraite de Saccharomyces cerevisiae.

4. Composition selon l'une des revendications 1, 2 ou 3, caractérisée en ce que ladite protéine de transfert est présente à raison de 0,0001 à 10 % en poids.

5. Composition selon la revendication 4, caractérisée en ce que ladite protéine de transfert est présente à raison de 0,001 à 5 % en poids.

6. Composition selon l'une quelconque des revendications 1 à 5, caractérisée en ce que la protéine de transfert est introduite sous forme de préparation la contenant, ayant une activité spécifique de transfert d'au moins 25 nM/mg/h.

7. Composition selon la revendication 6, caractérisée en ce que la protéine de transfert est introduite sous forme de préparation la contenant, ayant une activité spécifique de transfert d'au moins 250 nM/mg/h.

8. Composition selon l'une quelconque des revendications 1 à 7, caractérisée en ce qu'elle contient aussi un phospholipide ou un mélange de phospholipides.

9. Composition selon la revendication 8, caractérisée en ce que les phospholipides sont présents à raison de 0,001 à 50 % en poids.

10. Composition selon la revendication 9, caractérisée en ce que les phospholipides sont présents à raison de 0,01 à 20 % en poids.

11. Composition selon la revendication 8, caractérisée en ce que le ou les phospholipides associés aux protéines de transfert sont sous forme de liposomes ou de mélange de liposomes.

12. Composition selon la revendication 11, caractérisée en ce que les liposomes ou les mélanges de liposomes sont présents à raison de 0,01 à 50 % en poids.

13. Composition selon la revendication 12, caractérisée en ce que les liposomes ou les mélanges de liposomes sont présents à raison de 1 à 20 % en poids.

Office européen **RAPPORT DE RECHERCHE EUROPEENNE** Numero de la demande

des brevets

EP 92 40 0630

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,X | FR-A-2 631 236 (KARL HEINZ SCHMIDT) * Page 3, lignes 12-15,34-35; page 4, lignes 2-5,18-28,32-35; page 5, lignes 10-15; page 9, lignes 1-3; revendications 1,5,9,14 * | 1,8,11 | A 61 K 7/48 A 61 K 7/00 |
| D,Y | | 1,3,8, 11 | |
| D,Y | BIOCHEM. BIOPHYS. ACTA, vol. 794, 1984, pages 385-391, Elsevier Science Publishers B.V., Amsterdam, NL; G. DAUM et al.: "Phospholipid transfer in yeast. Isolation and partial characterization of a phospholipid transfer from yeast cytosol" * Page 385 * | 1,3,8, 11 | |

DOMAINES TECHNIQUES RECHERCHES (Int. Cl.5)

A 61 K

Le présent rapport a été établi pour toutes les revendications

| Lieu de la recherche | Date d'achèvement de la recherche | Examinateur |
|---|---|---|
| LA HAYE | 14-05-1992 | SIERRA GONZALEZ M.T. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)